# EUROPEAN PATENT SPECIFICATION

(11) **EP 1 680 060 B1**
(45) Date of publication and mention of the grant of the patent: **05.03.2008**
(21) Application number: 04789679.0
(22) Date of filing: 29.10.2004
(51) Int. Cl.: A61F 13/20

(54) **A HYGIENIC TAMPON AND AN ABSORBENT BODY USED IN THE FORMATION OF A TAMPON**
HYGIENISCHER TAMPON UND BEI DER BILDUNG EINES TAMPONS VERWENDETER SAUGFÄHIGER KÖRPER
TAMPON HYGIENIQUE ET CORPS ABSORBANT UTILISE DANS LA FORMATION D'UN TAMPON

(30) Priority: 31.10.2003 US 700743
(43) Date of publication of application: 19.07.2006
(73) Proprietor: JOHNSON & JOHNSON CONSUMER COMPANIES, INC., Skillman, NJ 08558 (US)
(72) Inventor: COSTA, Rogerio, 12606-310 Lorena - SP (BR)
(74) Representative: Mercer, Christopher Paul
(86) International application number: PCT/BR2004/000214
(87) International publication number: WO 2005/041833

(56) References cited:
- US-A- 2 306 406
- US-A- 2 425 004
- US-A- 2 455 925
- US-A- 2 499 414
- US-A- 4 340 055

## Description

### Field of the invention

The present invention is directed to a novel process to manufacture absorbent tampons that is versatile and useful to produce tampons and to novel tampons produced thereby.

### Background of the invention

There are known two types of hygienic tampons, those that need an applicator to be inserted in the vagina and those that can be digitally inserted.

Both tampons have problem concerning the insertion, removal and absorbency capacity, due to the particularities of vagina's anatomy.

The problems of absorbency capacity occur due to some situations, as follows:
tampons do not properly fit the vagina;
tampons have been compressed to such an extent that they have difficulties opening or expanding rapidly enough after initial insertion into a woman's vagina to absorb the initial volumes of body fluid which contacts them;
the tampon's shape may not effectively match the structure of the vagina. Hence, tampons do not contact with the whole vaginal wall, creating a possibility of leakage;
the tampon does not contain a sufficient amount of absorbent material at its insertion end to be able to absorb and distribute completely the body fluid that contacts it.

In its normal, collapsed state, a woman's vagina has a much wider dimension in its transverse plane than in its vertical plane. It is also well known that the area determined by vaginal cavity is minimum near the introitus and maximum near the cervix. It is also flaccid and has multiple folds and wrinkles which provide channels through which a significant portion of the menstrual fluids normally flow.

Hence, the tampon should have a shape that enables it to pass through the vaginal orifice without discomfort, and when once inside the vaginal cavity and beyond the restrictions of the orifice may occupy a volume such that it contacts substantially all of surface of the vaginal walls, particularly the mentioned folds and channels. Such a tampon should conform to the shape of the vaginal cavity or cause the vaginal cavity to take on a full shape, substantially opening these folds and channels.

Examples of patents that purport to disclose tampons capable of expanding radially can be seen in U.S. Patent Nos. 2,499,414; 3,618,605 and 3,834,389. Each of these patent disclose tampons that have at least two layers, requiring complex manufacturing processes. Each of the tampons also requires a certain degree of compression in order to be inserted into an applicator or the vagina. As a result, the tampon may initially be stiff. Upon insertion and exposure to bodily fluids, the tampon expands radially. This expansion may not always be uniform outwardly and may not completely contact the surrounding vaginal walls.

Patent US 2,306,406 purports to disclose a tampon that is formed from a flat blank in which the ends are gathered into forming a flower-like shape. Glycerine gluten or some other sizing material is impregnated into the cover and enables the tampon to hold it's flower-like shape. Upon exposure to fluid, the sizing material softens at the petal-like corners, which open to expose a cup-shaped interior. The procedure to make this tampon involves multiple steps and components that may affect the tampons absorption and comfort properties.

Patent 4,335,720 purports to disclose a catamenial tampon having a hollow core opening onto its insertion end and having radial slots at said end in communication with the hollow core.

Finally, patent US 4,294,253 discloses a tampon made from a flat layer of absorbent material folded into a cylindrical shape. A lacing string is wrapped around the flat absorbent body. The absorbent body is then folded and the edges welded together. The absorbent body is compressed until the final shape of a tampon is attained. While this advanced the art, it nonetheless provides further room for improvement, including comfort.

What is needed, therefore, is a tampon that is simple to make and also affords the user leakage protection.

### Objectives of the Invention

An objective of the present invention is to provide a novel process to manufacture absorbent tampons that is versatile and useful to produce a variety of tampon structures.

Another objective of the present invention is to provide a tampon having at least one structural element comprising at least one element that is bonded in a moisture-resistant manner to itself that is capable of keeping the desired shape of the tampon after same is compacted and pressed.

Yet another objective of the present invention is to provide a tampon that provides improved fit within the vaginal cavity, thus providing a greater and better containment of body exudates.

An objective of one particular embodiment of this invention is to provide a tampon that contains different amount of material in its upper portion, intermediate regions and lower portion, where the lower portion has a greater flexibility to enable the easy digital insertion of the tampon without the need of an applicator and the upper portion or the intermediate regions have a great amount of absorbent material to enable their absorption capacity.

An objective of one particular embodiment of the present invention is to provide a pre-expanded tampon that can be easily inserted in user's vaginal cavity and that does not substantially change its shape, even after absorption of body fluids.

Yet another objective of the present invention is to provide absorbent bodies useful to create the tampons described above.

### Brief Description of the Invention

The objectives of the invention are achieved by a tampon having a pursed-up matrix having a vertex and gathered edges. The pursed-up matrix has a structural element comprising at least one element that is bonded in a moisture-resistant manner to itself around a central longitudinal portion to form a stiffened core element. The pursed-up matrix also includes an absorbent body disposed about the core element.

The objectives of the invention are also achieved by a process for manufacturing a hygienic tampon wherein it comprises the following steps:
(i) positioning a first structural layer comprising bondable, preferably thermoplastic, material in facing relation to a first surface of an absorbent body to form a substantially flat matrix;
(ii) positioning the matrix on a template having an orifice therein;
(iii) applying heat to the first structural layer sufficient to bond to itself in a moisture-resistant manner to form a stiffened core element;
(iv) applying force to a central portion of the matrix to drive the matrix through the template to form a pursed-up matrix having a vertex and gathered edges; and
(v) cooling the pursed-up matrix to form an elongate absorbent structure.

### Brief Description of the Drawings

The present invention will be described in more details based on the example of an embodiment represented in the drawings. The figures show:
Figure 1 is a side plan view of an absorbent matrix useful to form a tampon according to the present invention;
Figure 2 is a top plan view of the embodiment of the matrix of Fig. 1;
Figure 3 is a side plan view of an alternative absorbent matrix useful to form a tampon according to the present invention;
Figure 4 is a bottom plan view of the alternative embodiment of Fig. 3;
Figure 5 is a side plan view of a tampon formed from the absorbent matrix of Fig. 3;
Figure 6 is a perspective view of the tampon of Fig. 5;
Figure 7 is a top plan view of the tampon of Fig. 5;
Figure 8 is a cross-section of along line 8-8 of Fig. 5;
Figure 9 is plan view of an alternative tampon according to the present invention prior to absorbing fluids; and
Figure 10 is a perspective view of the tampon of Fig. 9, after absorbing fluids;
Figure 11 is a top plan view of an alternative absorbent matrix useful to form a tampon according to the present invention
Figure 12 is a side plan view of an alternative absorbent matrix useful to form a tampon according to the present invention;
Figure 13 is a top plan view of the alternative embodiment of Fig. 12;
Figure 14 is a perspective view of a tampon formed from the absorbent matrix of Fig. 13;
Figure 15 is a side plan view of the tampon of Fig. 14;
Figure 16 is a cross-section of along line 16-16 of Fig. 15;
Figure 17 is schematic view of a process useful for manufacturing the tampon of the present invention; and
Figure 17A is a schematic view of the initial part of a process alternative to that of Fig. 17.

### Detailed Description of the Figures

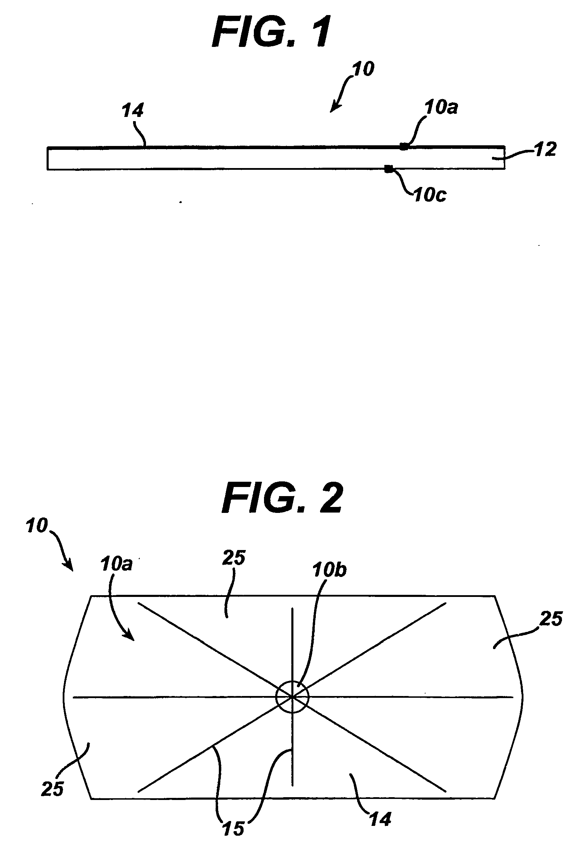

In accordance with the present invention, a hygienic tampon is formed from an absorbent matrix 10 as shown in Figs. 1 and 2. The matrix 10 comprises a substantially planar absorbent structure 12 and has an inner surface 10a, an outer surface 10c and a substantially centered region 10b. A first structural layer 14 is preferably disposed adjacent to the absorbent structure 12 at the inner surface 10a of the matrix 10. The matrix 10 may also have axially disposed embossments 15, which guide the folding of the matrix 10 as it is gathered and pursed up as described below. The pursed up matrix can form a tampon while the first structural layer is transformed into an internal stiffened core element by the application of energy, such as heat, and pressure. The stiffened core element can take the form of a "spinal column" or skeleton inside the tampon.

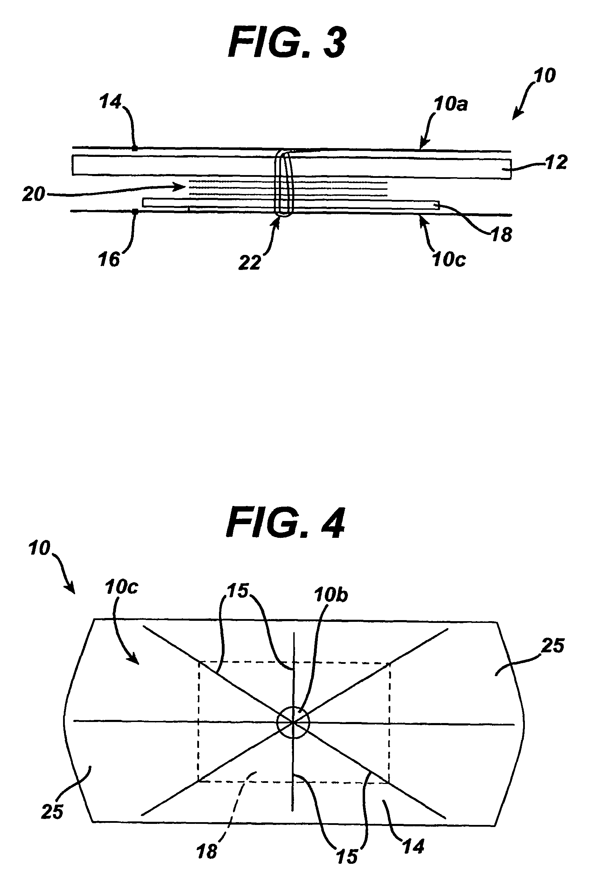

In one embodiment, illustrated in Figs. 3 and 4, the absorbent matrix 10 further includes an additional structural layer or cover layer 16 disposed adjacent the absorbent structure 12 at the outer surface 10c of the absorbent matrix 10. Thus, it is apparent that the first structural layer 14 and the cover layer 16 of this embodiment are wrapped around and substantially enclose the absorbent structure 12.

This embodiment can also include a containment element 18. The containment element 18 is substantially liquid-impermeable, is substantially smaller than the absorbent structure 12, and preferably, is attached to the absorbent structure 12 through an adhesive layer 20. The containment element 18 can help to prevent leakage of body exudates out of a tampon made from the absorbent matrix 10, even when the tampon is saturated. Additionally, the containment element 18 helps to keep the lower portion or withdrawal end of the tampon somewhat constricted, as it is not very expandable. Additionally, a string 22 can be attached to the absorbent matrix 10 to aid in the withdrawal of the resulting tampon from the user's body.

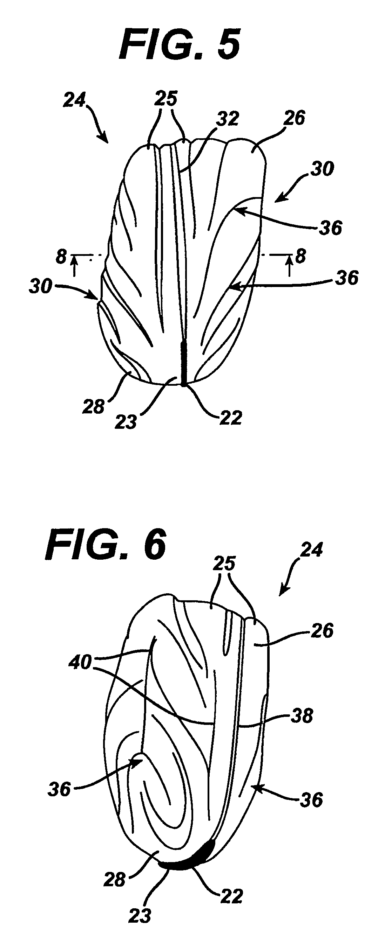

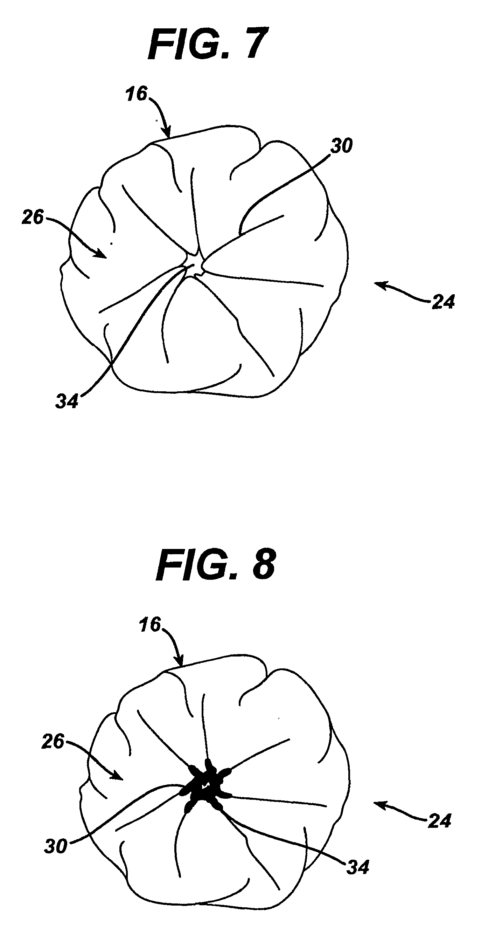

The absorbent matrix 10 can then be pursed up about a vertex 23 formed at its central region 10b by gathering the distal edges 25. The pursed up structure can form a tampon 24 as shown in Figs. 5 - 8 having an insertion end 26 and a withdrawal end 28. When pursed, the first structural layer 14 forms a stiffened core element 30 (shown in Fig. 8) about a central longitudinal axis 32 of the tampon 24. The stiffened core element 30 may define a void 34 at or essentially occlude the central longitudinal axis 32. In a preferred embodiment, the stiffened core element 30 defines a void 34 having an average diameter of less than about 1 mm, more preferably no discernible void. As shown in Fig. 5, the withdrawal string 22 is pressed into the withdrawal end 28. This string 22 can be extended, as desired, by the user. Because the tampon 24 as shown is formed from an elongate structure that is pursed up, two opposite side regions 36 are shown, separated by a parting line 38, which may not be readily apparent in the actual product. These side regions 36 may present a higher concentration of absorbent material.

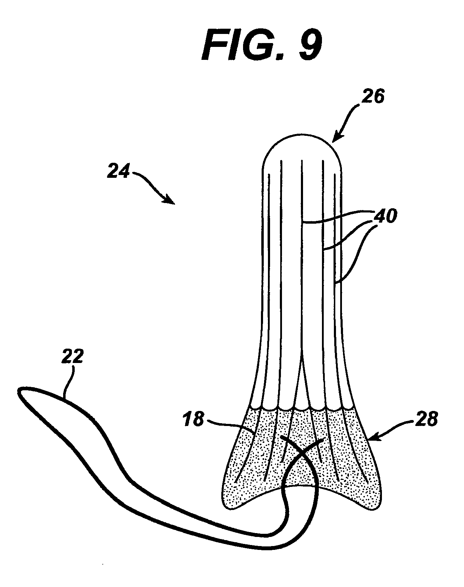

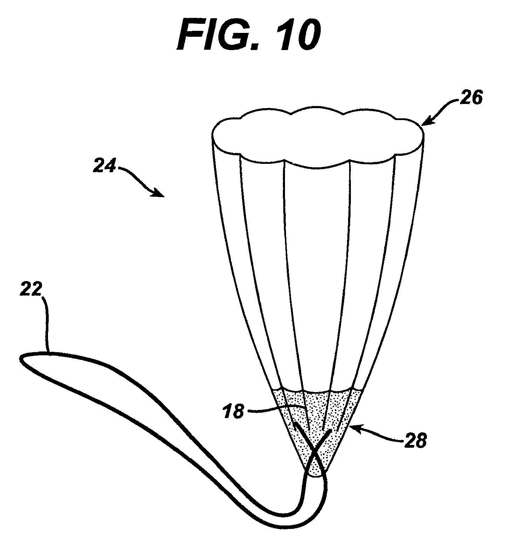

The tampon 24 as shown in Figs. 5-8 can also be further compressed, e.g., according to the processes described in Friese et al., US Pat. No. 6,310,269B1, and Leutwyler et al., US Pat. No. 5,911,712 to produce the tampon discussed below relating to Figs. 9 and 10. Tampons according to this embodiment may have an average density of at least about 0.3 g/cm3, more preferably, at least about 0.38 g/cm3, and most preferably, at least about 0.4 g/cm3.

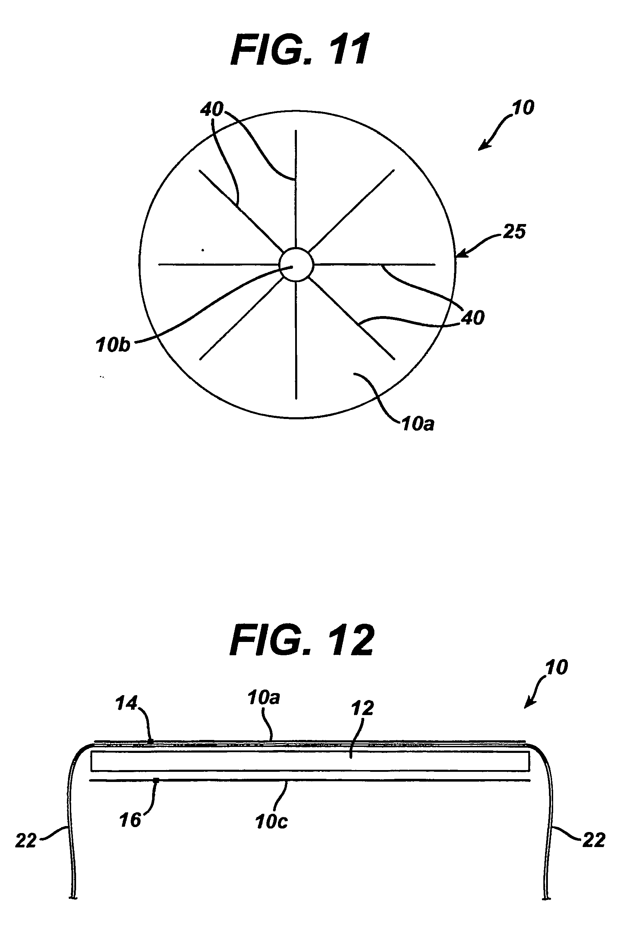

The foregoing discussion relates to an elongate absorbent matrix 10 that is pursed up about a vertex 23 located at the withdrawal end 28. One of ordinary skill in the art will recognize that the vertex could also form the insertion end of the tampon, and that the absorbent matrix can take on other shapes such as the circular matrix 10' illustrated in Fig. 11. Other shapes may include, without limitation, elliptical, oval, polygonal (e.g., octagonal and hexagonal).

As the withdrawal end 28 of the present embodiment comprises the vertex 23 of the pursed-up matrix, there is more material at the gathered distal edges 25. Thus, the insertion end 26 has a higher density than the withdrawal end 28, which mainly comprises the material present in the substantially central region 10b of the absorbent matrix 10 and the containment element 18.

This embodiment can incorporate a greater amount of absorbent material of the gathered edges 25 at the insertion end 26, and it can also further densify this absorbent material if the tip of the insertion end 26 is rounded. The insertion end 26 therefore can have a higher and a better capacity to absorb body exudates.

This higher absorption and containment of exudates at the insertion end 26 can make it possible for the tampon 24 to possess capacity to prevent the leaks caused by the saturation of the absorbent material and/or the excessive flow of exudates. It should also be stressed that a higher concentration of material in the insertion end 26 does not make it difficult for the user to insert the tampon 24, since said portion is tightly compressed to keep a suitable shape for insertion of the tampon 24.

On the other hand, since the withdrawal end 28 has a lower density and is less compacted than the insertion end 26, it is more flexible and soft. Thus, this end provides the user with greater comfort, and it may permit better conformity to the lower part of the user's vagina. This improved containment within the vagina may be enhanced by the presence of the containment element 18.

Another advantage attributed to the flexibility and lower density of the withdrawal end 28 is related to the better handling and hygiene provided thereby while the tampon 24 is inserted, that is, in view of the flexibility of the withdrawal end 28, when the tampon 24 is held while it is inserted, the user's finger deforms the trailing edge of the tampon 24 in such a way that her finger is substantially surrounded by the impermeable material of the containment element 18 as shown in Fig. 9.

When the tampon 24 is inserted in the vaginal cavity and contacts the dampness of the absorbed exudates, it can expand radially, but the expansion is not uniform along the length of the tampon.

As illustrated in Fig. 10, as the insertion end 26 absorbs the body exudates, it expands to better fill the user's vagina. This can help to reduce the passage of such exudates along the vaginal walls. Because the withdrawal end 28 has less absorbent material and incorporates the containment element 18, its radial expansion is significantly lower than that of the insertion end 26. This provides the tampon 24 a wedge shape. This shape can make it easier to remove the tampon 24 after use.

The use of an elongated absorbent matrix 10 shown in Fig. 3 and the resulting orientation of more absorbent material in the side regions 36 allow the tampon 24 to better contain the body exudates. This is because the tampon 24 can expand in a width-wise direction to form a final shape that is quite similar to the shape of the vaginal cavity. In this case, besides the higher amount of material in the first portion, the higher concentration of absorbent material at two intermediate opposite side regions 36, in a form of two ears, also increases absorbing capacity. This tampon 24 does not expand radially homogeneously, but forms a widened expanded tampon instead, that can cooperate better with the vaginal cavity.

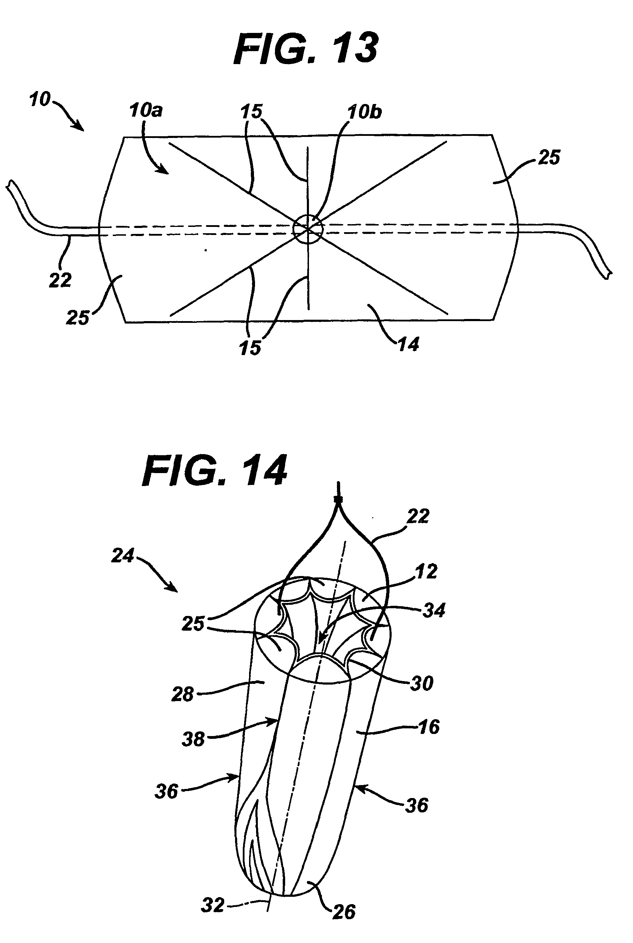

In another embodiment, illustrated in Figs. 12 and 13, the absorbent matrix 10 again includes an additional structural layer or cover layer 16 disposed adjacent the absorbent structure 12 at the outer surface 10c of the absorbent matrix 10. Thus, it is apparent that the first structural layer 14 and the cover layer 16 of this embodiment are wrapped around and substantially enclose the absorbent structure 12. Additionally, a string 22 can be attached to the absorbent matrix 10 to aid in the withdrawal of the resulting tampon from the user's body (shown here located below the first structural layer 14).

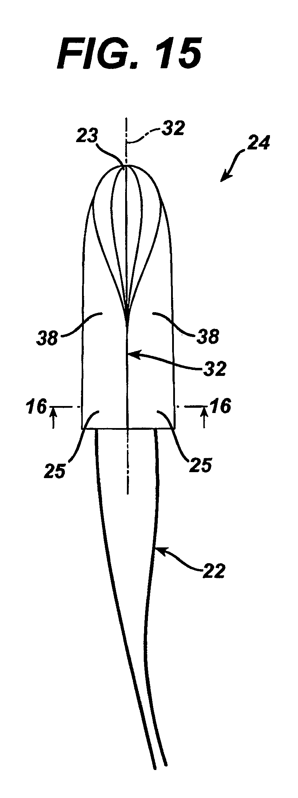

The absorbent matrix 10 of this embodiment can then be pursed up about a vertex 23 formed at its central region 10b by gathering the distal edges 25. The pursed up structure can form a tampon 24 as shown in Figs. 14 and 15 having an insertion end 26 and a withdrawal end 28. As shown in Fig. 14, the withdrawal string 22 extends from the withdrawal end 28. When pursed, the first structural layer 14 again forms a stiffened core element 30. In a preferred embodiment, the stiffened core element defines a void 34 about the central longitudinal axis 32 that can accommodate a user's finger to aid in the insertion of the tampon 24. Preferably, the void 34 has a diameter of at least about 5 mm.

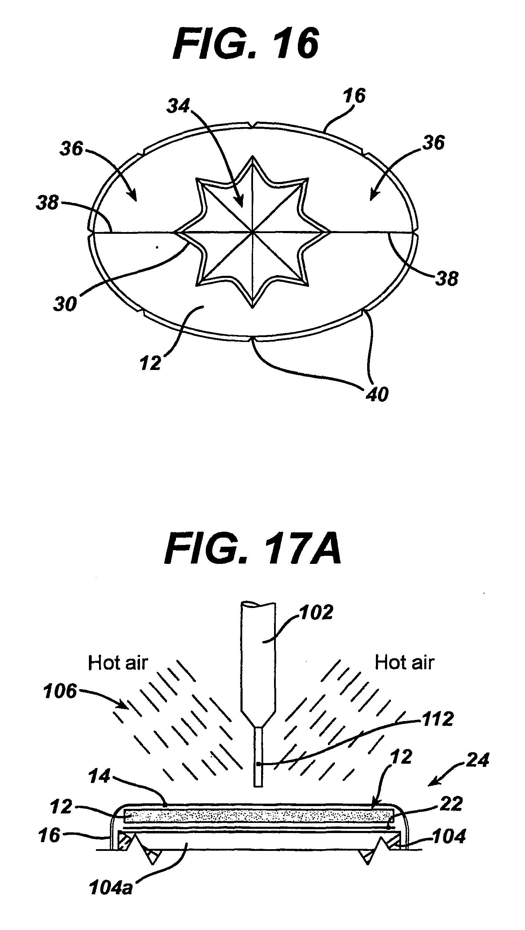

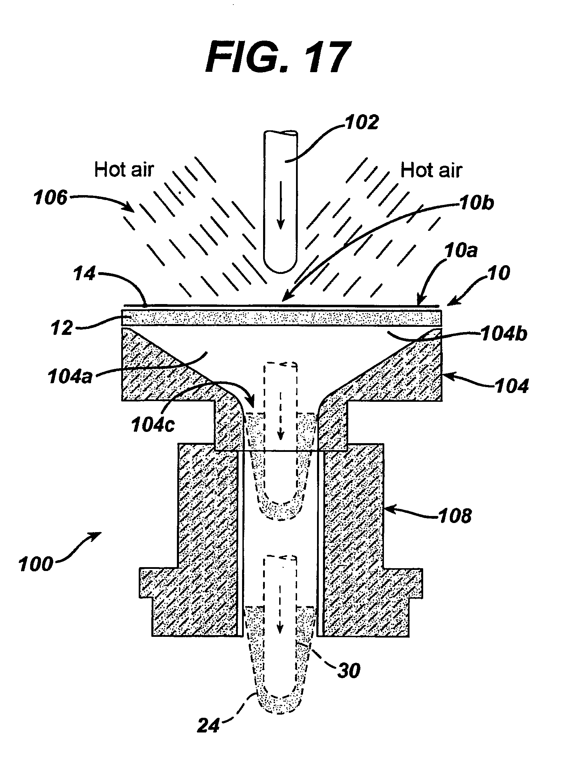

While this tampon 24 could be further compressed, it is preferred that the tampon 24 is stabilized in an expanded shape, for example the shape in which it exits the hollow forming tool 108 of Fig. 17.

Preferably, the tampon 24 presents a shape that does not vary significantly after its insertion in the user's vaginal cavity, and even after the contact with and absorption of the body exudates. Tampons according to this embodiment may have an average density of at least about 0.06 g/cm3. It is also preferred that the stabilized expanded tampon has a density of less than about 0.4 g/cm3.

The tampon 24 of this embodiment can also have two opposite side regions 36 separated by a parting line 38, which may not be readily apparent in the actual product. Again, these side regions 36 may present a higher concentration of absorbent material.

The foregoing discussion relates to an elongate absorbent matrix 10 that is pursed up about a vertex 23 located at the insertion end 26. One of ordinary skill in the art will recognize that the vertex could also form the insertion end of the tampon, and that the absorbent matrix can take on other shapes such as the circular matrix 10' illustrated in Fig. 11. Other shapes may include, without limitation, elliptical, oval, polygonal (e.g., octagonal and hexagonal).

While the tampon 24 shown in Figs. 9-13 has a substantially elliptical cross-section, it may also present other cross-sections, such as oval, cylindrical, octagonal among others, and while it is shown as generally straight, the tampon 24 can take on alternative shapes, including substantially curved along its longitudinal axis, in such a way that it assumes the aspect of the female vaginal cavity. These modifications can provide a tampon 24 as anatomically correct as possible.

The present embodiment of the tampon 24 (Figs. 12-16) can provide a pre-expanded product. In contrast to current commercial tampons, the present embodiment does not permit significant radial expansion and, therefore, has the same size and shape before and after its use. That is, the tampon 24 has a first initial shape and volume, as soon as it is manufactured, and a final shape and volume, after the use, that are substantially identical. Preferably, the volume of the tampon 24 after saturation with body fluids or exudates is less than 120% of the initial volume.

The properties of the pre-expanded tampon 24 of Figs. 12-16 can be varied through the manipulation of the components comprising the absorbent body (material and basis weight of absorbent structure 12, material and basis weight of the first and additional structural layers 14, 16, for example) and the process variables (hollow portion cross-section, force and velocity applied by the pushrod 102, energy applied, application time, and others). The variables can be balanced to provide a tampon that:
has a diameter that allows insertion into the vaginal cavity without excessive friction that could cause some discomfort;
has a stiffened core element (perhaps aided by an additional structural element) that is bonded in a moisture-resistant manner to itself in such a way that an expansion does not occurs, even after long exposure to body fluids, high humidity and forces applied due to user's movement;
may have a higher absorption capacity than conventional tampons using the same amount of material (or equivalent capacity with less material) to provide a more economical product; and
may ease the removal of the tampon, because rolled up layers are not formed that can spiral, as its shape does not change significantly, during use.

The void 34 makes it possible to insert the digital tampon of this invention more easily into the vaginal cavity. The void 34 comfortably and safely accommodates one finger of the user, confers to the tampon 24 a stability that makes possible the safe insertion, avoids the potential for the tampon to fall from the finger, avoids contact of the user's finger with the vagina or the external part of the tampon to avoid potential contamination.

Again, the foregoing discussion relates to an elongate absorbent matrix 10 that is pursed up about a vertex 23 located at the insertion end 26. One of ordinary skill in the art will recognize that the vertex could also form the withdrawal end of the tampon, and that the absorbent matrix can take on other shapes as discussed below.

Evidently, it may be foreseen the use of an applicator (not shown) to assist the insertion of the tampon 24 into the vaginal cavity. It may be foreseen a kit comprising the applicator and the tampon may be packed and sold as a unit.

The absorbent structure is preferably formed of absorbent materials including, without limitation, fiber, foam, hydrogels, wood pulp, superabsorbents, and the like. Preferred absorbent material for the present invention includes foam and fiber. Absorbent foams may include hydrophilic foams, foams which are readily wetted by aqueous fluids as well as foams in which the cell walls that form the foam themselves absorb fluid.

Preferred fibers employed in the formation of the absorbent body include regenerated cellulosic fiber, natural fibers and synthetic fibers. A useful, non-limiting list of useful absorbent body fibers includes natural fibers such as cotton, wood pulp, jute, and the like; and processed fibers such as regenerated cellulose, cellulose nitrate, cellulose acetate, rayon, polyester, polyvinyl alcohol, polyolefin, polyamine, polyamide, polyacrylonitrile, and the like. Other fibers in addition to the above fibers may be included to add desirable characteristics to the absorbent body. Preferably, the absorbent matrix fibers are rayon or cotton, and more preferably, the fibers are rayon. The fibers may have any useful cross-section.

Fiber cross-sections include multi-limbed and non-limbed. Multi-limbed, regenerated cellulosic fibers have been commercially available for a number of years. These fibers are known to possess increased specific absorbency over non-limbed fibers. A commercial example of these fibers is the Danufil^{®} VY multilimbed viscose rayon fibers available from Acordis UK Ltd., Spondon, England. These fibers are described in detail in Wilkes et al., US Pat. No. 5,458,835, the disclosure of which is hereby incorporated by reference.

Both the first structural layer 14 and any additional structural layer or cover layer 16 illustrated in Fig. 3 are constituted of material that is capable of being bonded in a moisture-resistant manner to itself to form a stiffened core element in the tampon. Useful materials include thermoplastic layers (such as films and fibrous webs or such elements having a thermoplastic powder applied thereto), adhesives, curable materials such cross-linkable materials, and the like. In the embodiment illustrated in Fig. 3, the first structural layer 14 is a permeable film or fibrous web comprising thermoplastic polymers. A representative, non-limiting list of useful thermoplastic polymers includes polyolefins such as polypropylene and polypropylene, polyesters such as polyethylene terephthalate, nylons, acrylates, and the like. Of course two or more of these materials may be combined or blended in a film, a web of different materials or a web of bi-component fibers (such as polyethylene-coated polyester fibers). In a preferred embodiment, the first structural layer 14 has more thermoplastic fibers than the cover layer 16.

Preferably, the first structural layer 14 and the cover layer 16 have a basis weight between about 9 and about 80 g/m2, and, more preferably, the preferred basis weight of the layers is between about 9 and about 15 g/m2. As mentioned above, the use of a web comprising thermoplastic fiber is not a limitative choice, but a particular embodiment among others.

Preferably, the containment element 18 is comprised of a substantially planar layer made of impermeable polymeric material such as a polymeric film, or even another hydrophobic material, such as a hydrophobic nonwoven fabric.

Adhesive layer 20 is preferably an adhesive tape or glue. Its purpose is to provide a greater and better containment of body exudates.

The tampon 24 of the embodiments generally described above can be made by gathering the distal edges 25 of the absorbent matrix 10 and pursing the absorbent matrix 10 about the central region 10b that forms the vertex 23. This can be achieved by applying force to the central region 10b, for example using a pushrod 102 while supporting outer lying regions of the matrix, including the distal edges 25 with a form 104. As shown in Fig. 14, the force is generally applied in a substantially perpendicular orientation to the substantially planar absorbent matrix 10. The transformation of the first structural layer 14 into a stiffened core element 30 is initiated by the application of energy 106, such as heat. The transformation of the first structural layer 14 is aided by the application of radial pressure. The radial pressure can be applied in a mechanical press in which pressing elements move into the product (radially, longitudinally, axially, or any combination of directions), during transit through a hollow forming tool 108, or any combination of one or more of these pressing mechanisms.

After exiting the hollow forming tool 108, the shape of the absorbent body 24 is maintained due to the transformation of the structural layer 14 into a stiffened core element 30. Preferably, the transformation included bonding the material of the first structural layer 14 to itself in a moisture-resistant manner to form the stiffened core element 30. Of course, the stiffened core element 30 can be similarly bonded to the absorbent structure 12.

Finally, the additional structural layer or cover layer 16 can also be deformed and bonded to itself and or the absorbent structure 12 as the absorbent matrix 10 is pursed up to form the tampon.

After the tampon 24 exits the hollow forming tool 108, it can be further processed. For example, it can be compressed in a conventional tampon-forming press, such as Friese et al., US Pat. No. 6,310,269B1, and

Leutwyler et al., US Pat. No. 5,911,712, the disclosures of which are herein incorporated by reference. The resulting tampon can have an insertion end 26 with a substantially rounded shape and longitudinal grooves 40 (e.g., 40 in Fig. 6 or Figs. 9 and 10).

Additionally or alternatively, the hollow forming tool 108 can be fitted with ridges 110 that form corresponding grooves (e.g., 40 in Fig. 6) in the tampon 24. These grooves help to improve the ability of the tampon 24 to maintain a stable shape and can help to increase the column strength of the tampon 24. The forming apparatus of Fig. 17 has two distinct regions. A first portion includes a form 104, onto which the absorbent matrix 10 of, e.g., Figs. 9 and 10 is placed. This form 104 has a truncated conical cavity 104a, having a large first opening 104b opposite a smaller second opening 104c. A second portion of the forming apparatus is a hollow forming tool 108 that follows the second opening 104c. It is preferred that this forming tool 108 determines the final shape of the tampon 24. In a particularly preferred embodiment, the hollow forming tool 108 has a substantially elliptical cross-section, but it may also present others cross section embodiments, such as oval, cylindrical, octagonal among others. The use of an elongated absorbent matrix 10 shown in Fig. 10 and the use of such an elliptical forming tool 108 can again provide more absorbent material in the side regions 36 allow the tampon 24 to better contain the body exudates. This is again because the tampon 24 can fill a relatively wide space, similar to the shape of the vaginal cavity. Still alternatively, the tube can be substantially curved, in such a way that it provides a tampon that is substantially curved along its longitudinal axis to assume the aspect of the female vaginal cavity.

While the pushrod 102 can have a substantially cylindrical shape as illustrated in Fig. 17, it may be preferable to include an extension 112 shown in Fig. 17A.

The foregoing process uses the application of energy to transform the first structural layer into a stiffened core element. Sources of energy can include, without limitation, thermal, ultrasonic, electromagnetic energy (such as infrared energy and microwave energy), chemical energy (such as a chemical reaction or the removal of a liquid carrier from a polymeric material), and the like. One preferred source of energy is thermal energy, such as hot air. This can be applied to the inner surface 10a of the absorbent matrix 10, preferably at a working temperature between 60o C and 250o C, more preferably between 160o and 180o C. Of course, the temperature can vary depending on the material which the first structural element comprises. Preferably in conjunction with a thermoplastic material, the working temperature corresponds to the point where the material starts to soften or even the melting point of the material. The choice of the softening point can be related to the velocity of cooling of the structure after its formation into a tampon.

As already mentioned, the thermoplastic material may comprise a permeable mesh or thermoplastic structure comprising, e.g., polypropylene or polyethylene-coated polyester fibers. In the case of coated fibers, the polyester provides structure to the web while the polyethylene melts to connect the fibers together in the web. The second kind of fiber is called bi-component fiber, since it contains two distinct polymers.

With the application of the hot air directly to the inner surface 10a of the absorbent matrix 10, all of the layers of the matrix are heated, especially, the first structural layer 14 that is disposed at the inner surface 10a. The thermoplastic fibers (or other material used, if the case) that comprise the first structural layer 14 start to get soft and deform due to application of heat. This results in the transformation of the first structural layer (adhering it to the absorbent structure 12, the string 22, and other adjacent elements) into the stiffened core element.

Preferably and especially so for the embodiment of Figs. 12-16, but not necessarily, energy, such as heat application can be applied to the forming tool 108 in a manner that can modify the additional structural layer to form an outer structural element to help to keep the desired shape of the tampon 24.

In addition, energy can be applied to the absorbent matrix while it is in the hollow forming tool 108 to further stabilize the form of the tampon.

While the pushrod 102 may move the absorbent matrix through the forming apparatus as heat 106 is applied to the inner surface of the absorbent matrix and/or the inner surface of forming apparatus, the process may be varied so that the application of heat can occur first, when then hot air is applied until a peak temperature is reached, for example between 60o C and 250°C, and then, as soon as the application of heat ceases, the absorbent matrix can be forced through the forming apparatus.

Tampons are generally categorized in two classes: applicator tampons and digital tampons, and a certain amount of dimensional stability is useful for each type of tampon. Applicator tampons use a relatively rigid device to contain and protect the tampon prior to use. To insert the tampon into a body cavity, the applicator is partially inserted into the body cavity, and the tampon can be expelled therefrom. In contrast, digital tampons do not have an applicator to help guide them into the body cavity and require sufficient column strength to allow insertion without using an applicator. This strength can be determined by securing one end of the tampon to the fixed plate of a Instron Universal Testing Machine, available from Instron Corporation, Canton, Massachusetts, USA. The moveable plate is brought to contact the opposite end of the tampon and is then set to compress the tampon at a rate of about 5 cm/minute. The force exerted on the tampon is measured continuously, and the point at which this force begins to fall instead of rise is the point at which the tampon buckles. The maximum force achieved is the tampon's column strength. Preferably, tampons of the present invention have a significant column strength, at least about 10 N. More preferably, the tampons have a column strength of at least about 20 N, and most preferably, they have a column strength of about 30 N to about 85 N. Tampons with a column strength that is too low do not have sufficient dimensional stability to maintain their basic structure during insertion as a digital tampon; tampons with a column strength which is too high can be perceived as being too stiff or hard to be comfortably inserted as a digital tampon.

In spite of the fact that an example of the preferred embodiment has been disclosed, it should be understood that the scope of the present invention encompasses other possible variations, being limited by the tenor of the accompanying claims, the possible equivalents being included.

## Claims

1. A tampon comprising a pursed-up matrix having a vertex and gathered edges, wherein the pursed-up matrix comprises
a structural element comprising at least one element that is bonded in a moisture-resistant manner to itself around a central longitudinal portion to form a stiffened core element; and
an absorbent body disposed about the core element.

2. The tampon of claim 1 wherein the tampon has a column strength of at least about 10 N.

3. The tampon of claim 1 wherein the stiffened core element defines a void at the central longitudinal portion.

4. The tampon of claim 3 wherein the void has an average diameter of less than about 1 mm.

5. The tampon of claim 1 wherein the absorbent body is substantially surrounded by a liquid-pervious cover.

6. The tampon of claim 1 wherein the tampon has an insertion end comprising the gathered edges of the pursed-up matrix and a withdrawal end comprising the vertex of the pursed-up matrix.

7. The tampon in accordance with claim 6 wherein the tampon has an average density of at least about 0.38 g/cm3.

8. The tampon in accordance with claim 6 wherein the tampon further comprises a withdrawal string extending from the withdrawal end.

9. The tampon in accordance with claim 6, which further comprises a containment element associated with the vertex of the pursed up matrix.

10. The tampon in accordance with claim 6, wherein the insertion end has a higher density than the withdrawal end.

11. The tampon in accordance with claim 10, wherein the withdrawal end is more flexible than the insertion end.

12. The tampon in accordance with claim 1 wherein the tampon has an insertion end comprising the vertex of the pursed-up matrix and withdrawal end comprising the gathered edges of the pursed-up matrix.

13. The tampon in accordance with claim 12 wherein the tampon has an average density of less than about 0.4 g/cm3.

14. The tampon in accordance with claim 12 wherein the tampon further comprises a withdrawal string extending outwardly from the withdrawal end.

15. The tampon in accordance with claim 12 wherein the tampon is capable of absorbing liquids and wherein the tampon has an initial volume and a volume after fluid saturation, and the volume after fluid saturation is less than 120% of the initial volume.

16. The tampon in accordance with claim 12 wherein the absorbent body is substantially surrounded by a liquid-pervious fibrous web cover, and the first structural element is formed from a fibrous web.

17. The tampon in accordance with claim 16 wherein the fibrous web from which the first structural element is formed has a higher basis weight than the fibrous web cover.

18. A process for manufacturing a hygienic tampon wherein it comprises the following steps:
(i) positioning a first structural layer comprising bondable material in facing relation to a first surface of an absorbent body to form a substantially flat matrix;
(ii) positioning the matrix on a template having an orifice therein;
(iii) applying energy to the first structural layer sufficient to bond to itself in a moisture-resistant manner to form a stiffened core element; and
(iv) applying force to a central portion of the matrix to drive the matrix through the template to form a pursed-up matrix having a vertex and gathered edges in the form of an elongate absorbent structure.

19. The manufacturing process in accordance with claim 18 wherein the stiffened core element is bonded around a central longitudinal portion of the pursed-up matrix.

20. The manufacturing process in accordance with claim 19 wherein the stiffened core element defines a void at the central longitudinal portion.

21. The manufacturing process in accordance with claim 18 further comprising the step of forming densified regions of the substantially flat matrix.

22. The manufacturing process in accordance with claim 21 wherein the densified regions radiate outwardly from the central portion of the matrix.

23. The manufacturing process in accordance with claim 18 which further comprises incorporating a withdrawal string with the matrix.

24. The manufacturing process in accordance with claim 18 which further comprises positioning a liquid-permeable cover layer in facing relation to a second surface of the absorbent body, opposite the first.

25. The manufacturing process in accordance with claim 18 wherein the heat application in step (iii) consists of applying hot air to the first structural layer.

26. The manufacturing process in accordance with claim 18, wherein step (iii) and step (iv) are carried out substantially simultaneously.

27. The manufacturing process in accordance with claim 18 further comprising compressing the absorbent structure to form a tampon having an average density of at least about 0.06 g/cm3.

28. The manufacturing process in accordance with claim 27 wherein the tampon has an average density of at least about 0.38 g/cm3.

29. The manufacturing process in accordance with claim 27 which further comprises the step of associating a containment element with the second surface of the central portion of the matrix.

30. The manufacturing process in accordance with claim 18 wherein the elongate absorbent structure is the tampon, and the tampon has an introduction end comprising the vertex of the pursed-up matrix.

31. The manufacturing process in accordance with claim 30, wherein a punch applies the force to the central portion of the matrix and deforms the first structural layer to form a void in the pursed-up matrix.

32. The manufacturing process in accordance with claim 30, wherein the energy is also applied to an inner surface of the template.

33. The manufacturing process in accordance with claim 18, wherein the energy comprises thermal energy.

34. The manufacturing process in accordance with claim 18, wherein the energy comprises ultrasonic energy.

35. The manufacturing process in accordance with claim 18, wherein the bondable material comprises a thermoplastic material.

## Patentansprüche

1. Tampon, umfassend eine zusammengezogene Matrix mit einem Scheitel und mit zusammengerafften Rändern, **dadurch gekennzeichnet, dass** die zusammengezogene Matrix umfasst:
ein strukturelles Element, das zumindest ein Element umfasst, das in einer feuchtigkeitsbeständigen Weise mit sich selbst rund um einen zentralen longitudinalen Abschnitt verbunden ist, um ein versteiftes Kernelement auszubilden; und
einen saugfähigen Körper, der um das Kernelement herum angeordnet ist.

2. Tampon nach Anspruch 1, **dadurch gekennzeichnet, dass** der Tampon eine Formfestigkeit von zumindest zirka 10 N aufweist.

3. Tampon nach Anspruch 1, **dadurch gekennzeichnet, dass** das versteifte Kernelement einen Hohlraum an dem zentralen longitudinalen Abschnitt begrenzt.

4. Tampon nach Anspruch 3, **dadurch gekennzeichnet, dass** der Hohlraum einen durchschnittlichen Durchmesser von weniger als zirka 1 mm aufweist.

5. Tampon nach Anspruch 1, **dadurch gekennzeichnet, dass** der saugfähige Körper im wesentlichen von einer flüssigkeitsdurchlässigen Abdeckung umgeben ist.

6. Tampon nach Anspruch 1, **dadurch gekennzeichnet, dass** der Tampon ein Einführungsende aufweist, welches die zusammengerafften Ränder der zusammengezogenen Matrix umfasst, und ein Rückholende, welches den Scheitel der zusammengezogenen Matrix umfasst.

7. Tampon nach Anspruch 6, **dadurch gekennzeichnet, dass** der Tampon eine durchschnittliche Dichte von zumindest zirka 0,38 g/cm³ aufweist.

8. Tampon nach Anspruch 6, **dadurch gekennzeichnet, dass** der Tampon weiterhin ein Rückholbändchen umfasst, das sich aus dem Rückholende heraus erstreckt.

9. Tampon nach Anspruch 6, welcher weiterhin ein Rückhalteelement umfasst, das mit dem Scheitel der zusammengezogenen Matrix in Verbindung steht.

10. Tampon nach Anspruch 6, **dadurch gekennzeichnet, dass** das Einführungsende eine größere Dichte aufweist als das Rückholende.

11. Tampon nach Anspruch 10, **dadurch gekennzeichnet, dass** das Rückholende flexibler ist als das Einführungsende.

12. Tampon nach Anspruch 1, **dadurch gekennzeichnet, dass** der Tampon ein Einführungsende aufweist, welches den Scheitel der zusammengezogenen Matrix umfasst, und ein Rückholende, welches die zusammengerafften Ränder der zusammengezogenen Matrix umfasst.

13. Tampon nach Anspruch 12, **dadurch gekennzeichnet, dass** der Tampon eine durchschnittliche Dichte von weniger als zirka 0,4 g/cm³ aufweist.

14. Tampon nach Anspruch 12, **dadurch gekennzeichnet, dass** der Tampon weiterhin ein Rückholbändchen umfasst, das sich nach außen vom Rückholende aus erstreckt.

15. Tampon nach Anspruch 12, **dadurch gekennzeichnet, dass** der Tampon in der Lage ist, Flüssigkeiten aufzusaugen und wobei der Tampon ein anfängliches Volumen und ein Volumen nach der Flüssigkeitssättigung aufweist, und das Volumen nach der Flüssigkeitssättigung weniger als 120 % des anfänglichen Volumens beträgt.

16. Tampon nach Anspruch 12, **dadurch gekennzeichnet, dass** der saugfähige Körper im wesentlichen von einer flüssigkeitsdurchlässigen Fasergewebeabdeckung umgeben ist und das erste strukturelle Element aus einem Fasergewebe ausgebildet ist.

17. Tampon nach Anspruch 16, **dadurch gekennzeichnet, dass** das Fasergewebe, aus welchem das erste strukturelle Element ausgebildet ist, ein höheres Basisgewicht aufweist als die Fasergewebeabdeckung.

18. Verfahren für die Herstellung eines hygienischen Tampons, **dadurch gekennzeichnet, dass** es die folgenden Schritte umfasst:
(i) Positionieren einer ersten strukturellen Lage, die verbindbares Material in einem einer ersten Oberfläche eines saugfähigen Körpers zugewandten Verhältnis umfasst, um eine im wesentlichen flache Matrix auszubilden;
(ii) Positionieren der Matrix auf einer Schablone, die eine Öffnung in derselben aufweist;
(iii) Anwenden von Energie auf die erste strukturelle Lage, die ausreicht, um diese mit sich selbst in einer feuchtigkeitsbeständigen Weise zu verbinden, um ein versteiftes Kernelement auszubilden; und
(iv) Anwenden von Kraft auf einen zentralen Abschnitt der Matrix, um die Matrix durch die Schablone hindurch zu treiben, um eine zusammengezogene Matrix auszubilden, die einen Scheitel und zusammengeraffte Ränder in Form einer länglichen saugfähigen Struktur aufweist.

19. Herstellungsverfahren nach Anspruch 18, **dadurch gekennzeichnet, dass** das versteifte Kernelement rund um einen zentralen longitudinalen Abschnitt der zusammengezogenen Matrix befestigt wird.

20. Herstellungsverfahren nach Anspruch 19, **dadurch gekennzeichnet, dass** das versteifte Kernelement einen Hohlraum am zentralen longitudinalen Abschnitt begrenzt.

21. Herstellungsverfahren nach Anspruch 18, das weiterhin den Schritt der Ausbildung von verdichteten Bereichen der im wesentlichen flachen Matrix umfasst.

22. Herstellungsverfahren nach Anspruch 21, **dadurch gekennzeichnet, dass** die verdichteten Bereiche sich vom zentralen Abschnitt der Matrix aus strahlenförmig nach außen ausbreiten.

23. Herstellungsverfahren nach Anspruch 18, welches weiterhin die Einfügung eines Rückholbändchens mit der Matrix umfasst.

24. Herstellungsverfahren nach Anspruch 18, das weiterhin das Positionieren einer flüssigkeitsdurchlässigen Decklage in einem einer zweiten Oberfläche des saugfähigen Körpers zugewandten Verhältnis, der ersten Oberfläche gegenüber liegend, umfasst.

25. Herstellungsverfahren nach Anspruch 18, **dadurch gekennzeichnet, dass** das Aufbringen von Wärme beim Schritt (iii) darin besteht, dass heiße Luft auf die erste strukturelle Lage aufgebracht wird.

26. Herstellungsverfahren nach Anspruch 18, **dadurch gekennzeichnet, dass** Schritt (iii) und Schritt (iv) im wesentlichen gleichzeitig durchgeführt werden.

27. Herstellungsverfahren nach Anspruch 18, das weiterhin das Komprimieren der saugfähigen Struktur für die Ausbildung eines Tampons mit einer durchschnittlichen Dichte von zumindest zirka 0,06 g/cm³ umfasst.

28. Herstellungsverfahren nach Anspruch 27, **dadurch gekennzeichnet, dass** der Tampon eine durchschnittliche Dichte von zumindest zirka 0,38 g/cm³ aufweist.

29. Herstellungsverfahren nach Anspruch 27, das weiterhin den Schritt des Verbindens eines Rückhalteelements mit der zweiten Oberfläche des zentralen Abschnitts der Matrix umfasst.

30. Herstellungsverfahren nach Anspruch 18, **dadurch gekennzeichnet, dass** die längliche saugfähige Struktur der Tampon ist und der Tampon ein Einführungsende hat, welches den Scheitel der zusammengezogenen Matrix umfasst.

31. Herstellungsverfahren nach Anspruch 30, **dadurch gekennzeichnet, dass** eine Stanze die Kraft auf den zentralen Abschnitt der Matrix aufbringt und die erste strukturelle Lage verformt, um einen Hohlraum in der zusammengezogenen Matrix auszubilden.

32. Herstellungsverfahren nach Anspruch 30, **dadurch gekennzeichnet, dass** die Energie ebenfalls auf eine innere Oberfläche der Schablone aufgebracht wird.

33. Herstellungsverfahren nach Anspruch 18, **dadurch gekennzeichnet, dass** die Energie Wärmeenergie umfasst.

34. Herstellungsverfahren nach Anspruch 18, **dadurch gekennzeichnet, dass** die Energie Ultraschallenergie umfasst.

35. Herstellungsverfahren nach Anspruch 18, **dadurch gekennzeichnet, dass** das verbindbare Material ein thermoplastisches Material umfasst.

## Revendications

1. Tampon comprenant une matrice froncée ayant un sommet et des bords rassemblés, dans lequel la matrice froncée comprend
◆ un élément structurel comprenant au moins un élément qui est lié sur lui-même autour d'une partie longitudinale centrale d'une manière résistant à l'humidité pour former un élément formant noyau renforcé ; et
◆ un corps absorbant disposé autour de l'élément formant noyau.

2. Tampon selon la revendication 1, dans lequel le tampon a une résistance en colonne d'au moins 10N environ.

3. Tampon selon la revendication 1, dans lequel l'élément formant noyau renforcé définit un vide au niveau de la partie longitudinale centrale.

4. Tampon selon la revendication 3, dans lequel le vide a un diamètre moyen inférieur à environ 1 mm.

5. Tampon selon la revendication 1, dans lequel le corps absorbant est sensiblement entouré d'une couverture perméable aux liquides.

6. Tampon selon la revendication 1, dans lequel le tampon a une extrémité d'insertion comprenant les bords rassemblés de la matrice froncée et une extrémité de retrait comprenant le sommet de la matrice froncée.

7. Tampon selon la revendication 6, dans lequel le tampon a une densité moyenne supérieure ou égale à environ 0,38 g/cm³.

8. Tampon selon la revendication 6, dans lequel le tampon comprend en outre un cordonnet de retrait s'étendant depuis d'extrémité de retrait.

9. Tampon selon la revendication 6, comprenant en outre un élément de rétention associé au sommet de la matrice froncée.

10. Tampon selon la revendication 6, dans lequel l'extrémité d'insertion a une densité supérieure à celle de l'extrémité de retrait.

11. Tampon selon la revendication 10, dans lequel l'extrémité de retrait est plus flexible que l'extrémité d'insertion.

12. Tampon selon la revendication 1, dans lequel le tampon a une extrémité d'insertion comprenant le sommet de la matrice froncée et une extrémité de retrait comprenant les bords rassemblés de la matrice froncée.

13. Tampon selon la revendication 12, dans lequel le tampon a une densité moyenne inférieure à environ 0,4 g/cm³.

14. Tampon selon la revendication 12, dans lequel le tampon comprend en outre un cordonnet de retrait s'étendant vers l'extérieur à partir de l'extrémité de retrait.

15. Tampon selon la revendication 12, dans lequel le tampon est apte à absorber des liquides et dans lequel le tampon a un volume initial et un volume suivant la saturation en fluides, et le volume suivant la saturation en fluides est inférieur à 120 % du volume initial.

16. Tampon selon la revendication 12, dans lequel le corps absorbant est sensiblement entouré d'une couverture en toile fibreuse perméable aux liquides, et le premier élément structurel est formé d'une toile fibreuse.

17. Tampon selon la revendication 16, dans lequel la toile fibreuse à partir de laquelle est formé le premier élément structurel a un grammage supérieur à la couverture en toile fibreuse.

18. Procédé de fabrication d'un tampon hygiénique, dans lequel le procédé comprend les étapes consistant à :
(i) positionner une première couche structurelle comprenant du matériau liant dans une relation en vis-à-vis avec une première surface d'un corps absorbant pour former une matrice sensiblement plate ;
(ii) positionner la matrice sur un gabarit ayant un orifice dans celui-ci ;
(iii) appliquer sur la première couche structurelle une énergie suffisante pour la lier à elle-même de manière résistant à l'humidité pour former un élément formant noyau renforcé ; et
(iv) appliquer une force à une partie centrale de la matrice pour entraîner la matrice à travers le gabarit pour former une matrice froncée ayant un sommet et des bords rassemblés en forme de structure absorbante allongée.

19. Procédé de fabrication selon la revendication 18, dans lequel l'élément formant noyau renforcé est lié autour d'une partie longitudinale centrale de la matrice froncée.

20. Procédé de fabrication selon la revendication 19, dans lequel l'élément formant noyau renforcé définit un vide au niveau de la partie longitudinale centrale.

21. Procédé de fabrication selon la revendication 18, comprenant en outre l'étape consistant à former des zones densifiées de la matrice sensiblement plate.

22. Procédé de fabrication selon la revendication 21, dans lequel les zones densifiées rayonnent vers l'extérieur à partir de la partie centrale de la matrice.

23. Procédé de fabrication selon la revendication 18, comprenant en outre l'étape consistant à intégrer un cordonnet de retrait dans la matrice.

24. Procédé de fabrication selon la revendication 18, comprenant en outre l'étape consistant à positionner une couche de couverture perméable aux liquides dans une relation en vis-à-vis avec une seconde surface du corps absorbant, opposée à la première.

25. Procédé de fabrication selon la revendication 18, dans lequel l'application de chaleur à l'étape (iii) consiste à appliquer de l'air chaud sur la première couche structurelle.

26. Procédé de fabrication selon la revendication 18, dans lequel l'étape (iii) et l'étape (iv) sont exécutées sensiblement simultanément.

27. Procédé de fabrication selon la revendication 18, comprenant en outre l'étape consistant à comprimer la structure absorbante pour former un tampon ayant une densité moyenne d'au moins environ 0,06 g/cm³.

28. Procédé de fabrication selon la revendication 27, dans lequel le tampon a une densité moyenne d'au moins environ 0,38 g/cm³.

29. Procédé de fabrication selon la revendication 27, comprenant en outre l'étape consistant à associer un élément de rétention à la seconde surface de la partie centrale de la matrice.

30. Procédé de fabrication selon la revendication 18, dans lequel la structure absorbante allongée est le tampon, et le tampon présente une extrémité d'introduction comprenant le sommet de la matrice froncée.

31. Procédé de fabrication selon la revendication 30, dans lequel un poinçon applique la force à la partie centrale de la matrice et déforme la première couche structurelle pour former un vide dans la matrice froncée.

32. Procédé de fabrication selon la revendication 30, dans lequel l'énergie est aussi appliquée à une surface interne du gabarit.

33. Procédé de fabrication selon la revendication 18, dans lequel l'énergie comprend de l'énergie thermique.

34. Procédé de fabrication selon la revendication 18, dans lequel l'énergie comprend de l'énergie ultrasonore.

35. Procédé de fabrication selon la revendication 18, dans lequel le matériau liant comprend une matière thermoplastique.
